# EUROPÄISCHE PATENTANMELDUNG

(11) **EP 2 456 072 A2**
(43) Veröffentlichungstag der Anmeldung: **23.05.2012**
(21) Anmeldenummer: 11188366.6
(22) Anmeldetag: 09.11.2011
(51) Int. Cl.: H03K 17/945

(54) **Fußschalter mit Fußerkennung**

(30) Priorität: 17.11.2010 DE 202010015472 U; 25.02.2011 DE 202011000441 U
(71) Anmelder: steute Schaltgeräte GmbH & Co. KG, 32584 Löhne (DE)
(72) Erfinder: Twellmann, Ralf, 31863 Obernkirchen (DE); Fischer, Nils, 32457 Porta Westfalica (DE)
(74) Vertreter: Habbel, Ludwig

(57) **Zusammenfassung**

Die Erfindung schlägt einen Fußschalter (1) vor, mit einem Gehäuse (5) und mit wenigstens einem per Fuß betätigbaren, als Schalter oder Taster ausgestalteten Betätigungsorgan (2), und einen Annäherungssensor (10,11), welcher den Raum vor dem Betätigungsorgan (2) erfassend angeordnet ist, und eine elektronische Schaltung, die einen Signalausgang aufweist und derart ausgestaltet ist, dass sie bei sensorisch detektierter Annäherung eines Fußes an das Betätigungsorgan (2) den Signalausgang ansteuert, wobei ein an diesen Signalausgang angeschlossener Signalgeber ein optisches und / oder akustisches Signal abgibt.

## Beschreibung

Die Erfindung betrifft einen Fußschalter nach dem Oberbegriff des Anspruchs 1.

Gattungsgemäße Fußschalter sind aus der Medizintechnik bekannt. Sie werden dort eingesetzt, um Geräte, wie beispielsweise Hochfrequenz-Chirurgiegeräte, Patientenliegen, Röntgengeräte, Lasergeräte u. dgl., zu steuern. Da der Arzt bei seiner Arbeit am Patienten die Hände nicht frei hat, steuert er die medizinischen Geräte mit Hilfe der Fußschalter. Dies hat weiterhin den Vorteil, dass die Hände des Arztes steril bleiben und er lediglich mit den Füßen die ggf. unsterilen Fußschalter berührt.

In der Praxis steht der Arzt häufig vor dem Problem, mit Hilfe von mehreren Fußschaltern mehrere medizinische Geräte steuern zu müssen. Insbesondere wenn die Arbeit am Patienten ein so hohes Maß an Konzentration vom Arzt verlangt, dass dieser den Blick nicht vom Patienten abwenden kann, ist er zur Ansteuerung der medizinischen Geräte ausschließlich auf die taktilen Reize angewiesen, wenn er nach unterschiedlichen Fußschaltern zum Ansteuern der jeweils unterschiedlichen medizinischen Geräte tastet. Fehlbedienungen, beispielsweise das nicht rechtzeitige Einschalten des gewünschten medizinischen Gerätes oder das versehentliche Einschalten eines zu dem Zeitpunkt nicht erwünschten medizinischen Gerätes können Gefahren für den Patienten und/oder für den Arzt und ggf. anwesende Arzthelfer bedeuten.

Der Erfindung liegt die Aufgabe zugrunde, einen gattungsgemäßen Fußschalter dahingehend zu verbessern, dass dieser auch ohne Blickkontakt zum Fußschalter eine eindeutige Identifizierung des jeweiligen Fußschalters vor dessen Betätigung ermöglicht.

Diese Aufgabe wird durch einen Fußschalter mit den Merkmalen des Anspruchs 1 gelöst.

Die Erfindung schlägt mit anderen Worten vor, dem Betätigungsorgan des Fußschalters einen Annäherungssensor vorzuschalten, so dass die Anwesenheit des Fußes detektiert wird, bevor die eigentliche Aktion ausgelöst werden kann, die bei Betätigung des Betätigungsorgans vorgesehen ist, beispielsweise die Ansteuerung des an diesen Fußschalter angeschlossenen medizinischen Gerätes. Dabei ist es unerheblich, ob das Betätigungsorgan eine analoge oder digitale Funktion hat. Der Fußschalter weist eine elektronische Schaltung auf, die mit dem Annäherungssensor wirksam verbunden ist und die einen Signalausgang aufweist. Wenn sensorisch die Annäherung des Fußes an das Betätigungsorgan detektiert wird, so wird mittels der elektronischen Schaltung der Signalausgang automatisch angesteuert.

Ein an diesen Signalausgang angeschlossener Signalgeber gibt daraufhin ein optisches und/oder akustisches Signal ab. Auf diese Weise kann anhand des Signals erkannt werden, dass dieser Fußschalter betätigt wird, und dass eine weitere Bewegung des Fußes zur Aktivierung des Betätigungsorgans führen wird. Wenn mehrere Fußschalter vorschlagsgemäß ausgestaltet sind und sich ihre Signale voneinander unterscheiden, kann allein anhand dieser Signalisierung bereits vor Betätigung des jeweiligen Betätigungsorgans der Arzt darauf aufmerksam gemacht werden, welchen Fußschalter er momentan bedient und welches medizinische Gerät bei weiterer Betätigung des Fußschalters und bei Betätigung des Betätigungsorgans angesteuert werden wird. Auf diese Weise wird der Anwender vor einer Fehlbedienung des Fußschalters und vor einer versehentlichen Fehlbedienung eines medizinischen Gerätes gewarnt.

Der Annäherungssensor kann beispielsweise als Drahtbügel o. dgl. ausgestaltet sein, der federbelastet und beweglich ist, so dass durch Kontakt mit einem derartigen Bügel die Signalisierung ausgelöst werden kann. Vorteilhaft kann allerdings vorgesehen sein, dass der Annäherungssensor als berührungsloser Sensor ausgestaltet ist, so dass die Betätigung des eigentlichen Betätigungsorgans für den Anwender, der bereits an bestimmte Fußschalter gewöhnt ist, unverändert bleiben kann und keine zusätzlichen taktilen Reize, mechanischen Widerstände o. dgl. zu überwinden sind.

So kann beispielsweise der Annäherungssensor als Lichtschranke ausgestaltet sein, als kapazitiver, Infrarot- oder Ultraschallsensor, so dass jeweils auch glattflächige Oberflächen des Fußschalters ermöglicht werden, die eine einfache und zuverlässige Reinigung des Fußschalters gestatten.

Vorteilhaft kann der Signalgeber innerhalb des Fußschalters selbst angeordnet sein und ein akustisches Signal abgeben. Auf diese Weise ist die Signalisierung auch dann sichergestellt, wenn der Anwender nicht auf ein Display o. dgl., und insbesondere nicht auf den Fußschalter selbst während seiner Tätigkeit blicken möchte.

Alternativ dazu kann vorgesehen sein, dass der Signalgeber außerhalb des Fußschalters angeordnet ist und mit dem Signalausgang des Fußschalters übertragungswirksam verbunden werden kann. So kann beispielsweise ein an einem medizinischen Gerät ohnehin vorgesehener Monitor eine Warnleuchte o. dgl. als Signalgeber genutzt werden oder es kann eigens ein Bildschirm vorgesehen sein, an dem mehrere vorschlagsgemäß ausgestaltete Fußschalter angeschlossen sind, so dass auf diesem Bildschirm angezeigt werden kann, welcher Fußschalter jeweils betätigt wird oder kurz vor seiner Betätigung steht, an welchem Fußschalter also der Annäherungssensor die Anwesenheit eines Fußes detektiert hat.

Vorteilhaft können bei komplexer aufgebauten Fußschaltern, die mehrere Betätigungsorgane aufweisen, auch mehrere Annäherungssensoren vorgesehen sein, so dass zumindest Betätigungsorgane, die ähnlich aufgebaut sind und daher ähnlich zu betätigen sind und ähnlich taktile Reize dem Anwender vermitteln, voneinander unterschieden werden können. Dementsprechend sind mehreren Betätigungsorganen eines solchen Fußschalters jeweils ein eigener Annäherungssensor zugeordnet sowie eine elektronische Schaltung, welche die Signale der Annäherungssensoren auswertet und ein Signalgeber, der entweder je nach aktiviertem Sensor unterschiedliche Signale ausgibt, oder wobei jedem überwachten Betätigungsorgan ein eigener, unterschiedlich von anderen ausgestalteter Signalgeber zugeordnet ist.

Im einfachsten Fall jedoch, wenn sämtlichen Betätigungsorganen desselben Fußchalters stets dasselbe Signal zugeordnet ist, wird zumindest zuverlässig die Betätigung jedes Betätigungsorgans dieses Fußschalters durch die Annäherungssensoren überwacht, so dass dann eine Signalisierung erfolgen kann, die für sämtliche Betätigungsorgane dieses Fußschalters gleich ist und eine Unterscheidung von einer Betätigung eines anderen Fußschalters ermöglicht. Die Unterscheidung der einzelnen Betätigungsorgane innerhalb desselben Fußschalters kann dann in an sich bekannter Weise durch unterschiedliche Ausgestaltung der einzelnen Betätigungsorgane oder durch unterschiedliche Ausgestaltung des Gehäuses in der Umgebung der einzelnen Betätigungsorgane erfolgen, so dass die schon erwähnten taktilen Reize für die unterschiedlichen Betätigungsorgane desselben Fußschalters unterschiedlich sind und dem Anwender die erforderliche Unterscheidung ermöglichen.

Vorteilhaft kann das Betätigungsorgan von so genannten Sperrelementen umgeben sein, so dass das Betätigungsorgan nicht aus beliebigen Richtungen zugänglich ist. Auf diese Weise ergibt sich ein regelrechter Annäherungskorridor für die Betätigung des Betätigungsorgans, so dass auf diese Weise besonders zuverlässig die Überwachung des Betätigungsorgans mit Hilfe des Annäherungssensors erfolgen kann, indem dieser seinen Erfassungsbereich innerhalb des Annäherungskorridors hat.

Vorteilhaft kann eine besonders preisgünstige Ausgestaltung des Fußschalters dadurch erfolgen, dass das Gehäuse keine zusätzlichen Elemente, wie Sperrelemente oder Halterungen für die Annäherungssensoren o. dgl. benötigt, indem vielmehr das Betätigungsorgan selbst weiterentwickelt wird und derart ausgestaltet ist, dass es vor der Ansteuerung des angeschlossenen medizinischen Gerätes zunächst einen so genannten Leerweg aufweist, also einen Betätigungsweg, der noch nicht zum Ansteuern des angeschlossenen medizinischen Gerätes führt, sondern der als Bewegung des Betätigungsorgans sensorisch erfasst wird, so dass in diesem Bereich des Leerwegs der Annäherungssensor verwirklicht ist, der zu der entsprechenden Signalisierung führt.

Vorteilhaft können bei Ansteuerung mehrerer medizinischer Geräte mehrere vorschlagsgemäße Fußschalter vorgesehen sein, wobei diese Anordnung von mehreren Fußschaltern eine klare Identifizierung der unterschiedlichen medizinischen Geräte dadurch ermöglicht, dass jedem dieser Fußschalter ein unterschiedliches Signal zugeordnet ist.

Vorteilhaft kann die Art des Signals am Fußschalter selbst eingestellt werden, indem die elektronische Schaltung bzw. der Signalgeber dementsprechend einstellbar ist. Die Art des Signals kann aus mehreren unterschiedlichen Signalarten ausgewählt werden, beispielsweise bei akustischen Signalen in Form unterschiedlicher Tonhöhen oder unterschiedlicher Tonfolgen.

Ein Ausführungsbeispiel der Erfindung wird anhand der rein schematischen Darstellungen näher erläutert. Dabei zeigt
- Fig. 1: eine perspektivische Ansicht von oben auf einen Fußschalter, und
- Fig. 2: eine Ansicht wie in Fig. 1, wobei zur Betätigung eines Betätigungsorgans der Fuß eines Anwenders durch einen schematisch angedeuteten Schuh symbolisiert ist.

In den Zeichnungen ist mit 1 insgesamt ein Fußschalter bezeichnet, der zwei Betätigungsorgane 2 in Form von Tastern bzw. Schaltern aufweist. Die Betätigungsorgane 2 sind um jeweils eine Schwenkachse 3 beweglich gelagert und mit dem Fuß betätigbar, wenn der Fußschalter 1 auf einem Untergrund wie dem Boden eines Operationssaals o. dgl. aufliegt.

Die Betätigungsorgane 2 sind jeweils von Sperrelementen 4 umgeben, die von einem Gehäuse 5 des Fußhalters 1 gebildet werden. Die Sperrelemente 4 sind als zwei erhöhte, seitliche Rippen 6 und als ein mittlerer Trennsteg 7 ausgestaltet, wobei der Trennsteg 7 durch einen Aufsatz 8 eine zusätzlich vergrößerte Höhe aufweist. Mit Hilfe der Sperrelemente 4 ist verhindert, dass sich der Fuß des Anwenders den Betätigungsorganen 2 von der Seite her nähern kann. Vielmehr muss sich der Fuß des Anwenders jeweils von vorn bzw. von oben, also innerhalb eines durch die Sperrelemente 4 vorgegebenen Annäherungskorridors, dem jeweiligen Betätigungsorgan 2 nähern.

Innerhalb jedes dieser vorgegebenen Annäherungskorridore sind Erfassungsbereiche 9 von Annäherungssensoren 10 und 11 vorgesehen. Jeweils zwei 10 sind im Aufsatz 8 angeordnet sowie jeweils ein Annäherungssensor 11 in einer Rippe 6, wobei die Annäherungssensoren jeweils als Lichtschranke ausgestaltet und jeweils paarweise in Art einer Lichtschranken-Durchlichtvariante angeordnet sind. Die Annäherungssensoren 10 und 11 strahlen dabei durch den jeweiligen Erfassungsbereich 9 und sichern somit, über das jeweilige Betätigungsorgan 2 hinweg, den Annäherungskorridor oberhalb des jeweiligen Betätigungsorgans 2.

Abweichend von diesem Ausführungsbeispiel kann vorgesehen sein, dass die Annäherungssensoren 10 im Aufsatz 8 jeweils durch den Erfassungsbereich 9 eines kompletten Annäherungskorridors strahlen, also über die gesamte Breite eines Betätigungsorgans 2, und zwar bis zu einem gegenüberliegend in einer Rippe 6 angeordneten Reflektor, der in diesem Fall mit 11 gekennzeichnet wäre. Der Reflektor 11 reflektiert bei diesem Ausführungsbeispiel das Licht zum Annäherungssensor 10. Der Annäherungssensor 10 umfasst bei dieser Ausgestaltung also sowohl Licht aussendende als auch Licht empfangende Elemente, so dass eine vereinfachte Kabelführung ermöglicht ist, die in den Aufsatz 8 des Trennstegs 7 führt und dort zu beiden Annäherungssensoren 10.

Bei dem dargestellten Ausführungsbeispiel sind die Annäherungssensoren 10 und 11 im Gehäuse 5 des Fußschalters 1 angeordnet. Abweichend davon können auch anders ausgestaltete Annäherungssensoren Anwendung finden bzw. die Annäherungssensoren an anderer Stelle angeordnet sein. Beispielsweise kann im Betätigungsorgan 2 selbst ein Annäherungssensor verwirklicht sein, beispielsweise in Form eines kapazitiven Sensors, so dass bei Annäherung eines Fußes an das Betätigungsorgan 2 dieser Annäherungssensor aktiviert wird.

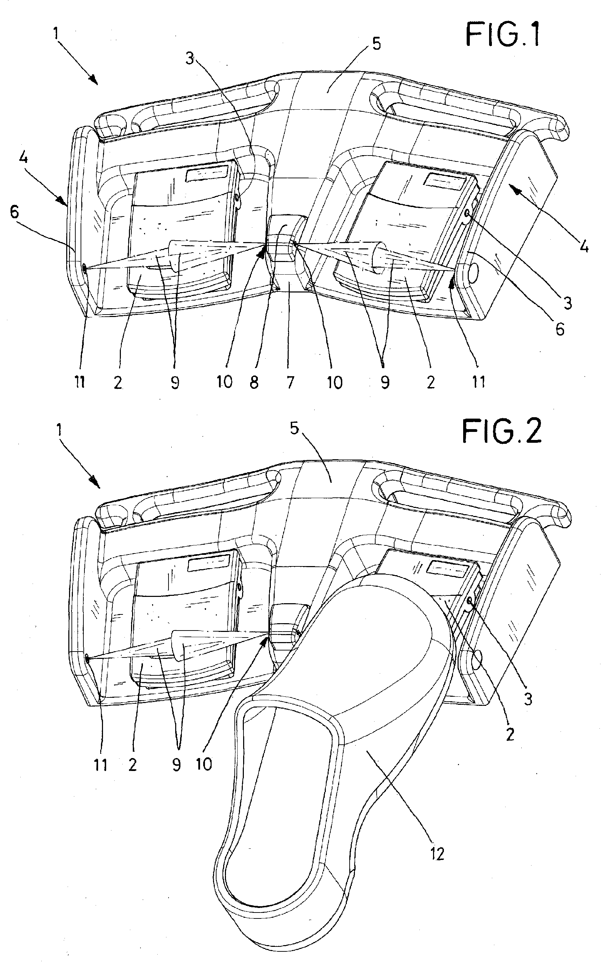

Fig. 2 zeigt den Fußschalter 1 von Fig. 1, wobei sich ein Schuh 12 im Erfassungsbereich 9 des rechten Betätigungsorgans 2 befindet, noch bevor der Schuh 12 das Betätigungsorgan 2 berührt. Eine im Fußschalter 1 vorgesehene bzw. dem Fußschalter 1 zugeordnete elektronische Schaltung ist mit den Annäherungssensoren 10 und 11 dieses rechten Betätigungsorgans 2 wirksam verbunden und aktiviert einen Signalgeber, wenn der Annäherungssensor 10 bzw. 11 die Anwesenheit des Schuhs 12 erfasst. Das daraufhin erzeugte Signal unterscheidet sich von einem Signal, welches abgegeben wird, wenn der Schuh 12 in den Erfassungsbereich 9 des linken Betätigungsorgans 2 gerät, so dass der Anwender durch die jeweilige Signalisierung noch vor Betätigung des jeweiligen Betätigungsorgans 2 einen Hinweis darauf erhält, welche Funktion eines an den Fußschalter 1 angeschlossenen Gerätes er demnächst auslösen wird.

## Patentansprüche

1. Fußschalter, mit einem Gehäuse und mit wenigstens einem per Fuß betätigbaren, als Schalter oder Taster ausgestalteten Betätigungsorgan,
**gekennzeichnet durch**
einen Annäherungssensor (10), welcher den Raum vor dem Betätigungsorgan (2) erfassend angeordnet ist, und eine elektronische Schaltung, die einen Signalausgang aufweist und derart ausgestaltet ist, dass sie bei sensorisch detektierter Annäherung eines Fußes an das Betätigungsorgan (2) den Signalausgang ansteuert, derart, dass ein an diesen Signalausgang angeschlossener Signalgeber ein optisches und / oder akustisches Signal abgibt.

2. Fußschatler nach Anspruch 1,
**dadurch gekennzeichnet,**
**dass** der Annäherungssensor (10) als berührungsloser Sensor ausgestaltet ist.

3. Fußschalter nach Anspruch 2,
**dadurch gekennzeichnet,**
**dass** der Annäherungssensor (10) als Lichtschranke ausgestaltet ist.

4. Fußschalter nach Anspruch 2,
**dadurch gekennzeichnet,**
**dass** der Annäherungssensor (10) als kapazitiver Sensor ausgestaltet ist.

5. Fußschalter nach Anspruch 2,
**dadurch gekennzeichnet,**
**dass** der Annäherungssensor (10) als Ultraschallsensor ausgestaltet ist.

6. Fußschalter nach Anspruch 2,
**dadurch gekennzeichnet,**
**dass** der Annäherungssensor (10) als Infrarotsensor ausgestaltet ist.

7. Fußschalter nach einem der vorhergehenden Ansprüche,
**dadurch gekennzeichnet,**
**dass** der Signalgeber innerhalb des Fußschalters (1) angeordnet und ein akustisches Signal abgebend ausgestaltet ist.

8. Fußschalter nach einem der Ansprüche 1 bis 6,
**dadurch gekennzeichnet,**
**dass** der Signalgeber außerhalb des Fußschalters (1) angeordnet und mit dem Signalausgang übertragungswirksam verbindbar ist.

9. Fußschalter nach Anspruch 7,
**dadurch gekennzeichnet,**
**dass** der Signalausgang des Fußschalters (1) an ein medizinisches Anzeigegerät angepasst ist, welches einen Signalgeber aufweist, derart, dass der Signalgeber des an den Signalausgang des Fußschalters (1) angeschlossenen medizinischen Anzeigegeräts das optische und / oder akustische Signal abgibt.

10. Fußschalter nach einem der vorhergehenden Ansprüche,
**gekennzeichnet durch**
mehrere Betätigungsorgane (2), wobei den Betätigungsorganen (2) jeweils ein Annäherungssensor (10), eine elektronische Schaltung und ein Signalgeber zugeordnet ist.

11. Fußschalter nach Anspruch 9,
**dadurch gekennzeichnet,**
**dass** jedem der mehreren Betätigungsorgane (2) ein unterschiedliches Signal zugeordnet ist.

12. Fußschalter nach einem der vorhergehenden Ansprüche,
**dadurch gekennzeichnet,**
**dass** das Betätigungsorgan (2) von Sperrelementen (4) umgeben ist, derart, dass der Zugang zu dem Betätigungsorgan (2) nur innerhalb eines von den Sperrelementen (4) freien Annäherungskorridors ermöglicht ist.

13. Fußschalter nach einem der vorhergehenden Ansprüche,
**dadurch gekennzeichnet,**
**dass** der Annäherungssensor (10) innerhalb des Betätigungsorgans (2) vorgesehen ist, derart, dass das Betätigungsorgan (2) zunächst einen als Leerweg bezeichneten Betätigungsweg aufweist, der zur Aktivierung des Annäherungssensors (10) dient.

14. Fußschalter nach einem der vorhergehenden Ansprüche,
**dadurch gekennzeichnet,**
**dass** die elektronische Schaltung und / oder der Signalgeber einstellbar ist, derart, dass die Art des Signals aus mehreren unterschiedlichen Signalarten wählbar ist.

15. Anordnung von mehreren Fußschaltern (1), die jeweils nach einem der vorhergehenden Ansprüche ausgestaltet sind, wobei jedem der mehreren Fußschalter (1) ein unterschiedliches Signal zugeordnet ist.
